# EUROPEAN PATENT SPECIFICATION

(11) **EP 0 425 783 B1**
(45) Date of publication and mention of the grant of the patent: **06.03.1996**
(21) Application number: 90116070.5
(22) Date of filing: 22.08.1990
(51) Int. Cl.: A61B 17/56, A61F 2/44

(54) **Pedicle screw clamp**
Klemme für eine Pedikelschraube
Pince pour une vis pédiculaire

(30) Priority: 30.10.1989 US 428907
(43) Date of publication of application: 08.05.1991
(73) Proprietor: Synthes AG, Chur, CH-7002 Chur (CH)
(72) Inventor: Frigg, Robert, CH-7270 Davos-Dorf (CH)
(74) Representative: Lusuardi, Werther Giovanni, Dr.

(56) References cited:
- EP-A- 0 242 708
- EP-A- 0 330 881
- DE-B- 2 834 891
- FR-A- 2 615 095
- US-A- 4 433 676
- US-A- 4 771 767

## Description

This invention relates to an osteosynthetic clamp according to the preamble of claim 1 and a fixation assembly according to the preamble of claim 16.

Pedicle screws held by clamps in osteosynthetic assemblies are one type of implant used for treating spinal injuries and deformities. In one common treatment the pedicle screws are driven into the pedicles of vertebrae above and below the injured vertebra or vertebrae. A supporting rod is attached to the pedicle screws, for example, by clamps or by threading it through slots in the pedicle screws. The supporting rod holds the spinal column approximately in its desired alignment, thereby relieving pressure on the injured vertebra or vertebrae and permitting it to heal and regain its natural conformation.

The German DE-AS 28.34.891 SCHLÄPFER discloses a spinal fixation device which allows to adjust the distance between a pair of fixation screws and the clamp holding them. This known fixation device, however, is rather limited in its general adaptability to the surgical situations that may be encountered.

First it can only be used as an external fixator (if it would be applied internally the spinous process would have to be removed) and second it is designed as a bilateral device i.e. it needs always a pair of two fixation screws which necessarily must lie in the same horizontal plane of each vertebra to be fixed with the screws. As a consequence of the latter requirement the two screws of each horizontal plane can only be moved simultaneously with relation to the clamp. Furthermore this known device necessitates two longitudinal rods lying in the sagittal plane in order to transmit forces between different vertebrae and accordingly the clamp is designed to receive two ball joints for accommodating the two rods. As a consequence it is not possible to effect a derotation of the vertebrae.

The US 4,771,767 STEFFEE discloses a spinal fixation device where the axis of the longitudinal bar and of the pedical screws are positioned in different planes but have a constant distance from each other. It is not possible, therefore, to position the two axis freely in space as desired.

As noted, clamps may be used to connect rigidly the part of the pedicle screw protruding from the vertebra to a spinal support rod. In most of these known clamps the pedicle screw and the supporting rod are arranged in the same plane allowing no adjustment to anatomical requirements.

In another known type of pedicle screw clamp (according to AT-B 387.710 SULZER) the central axis of the pedicle screw and the central axis of the support rod are located in different planes but still maintained at a fixed, non-adjustable distance, again preventing the surgeon to adapt the clamp to anatomical needs.
Furthermore these known pedicle screw clamps do not permit relative angular adjustment of the pedicle screw and the support rod. Thus, current clamps do not allow sufficient adjustment to the specific alignment required by each patient's need.

The invention as claimed is intended to remedy these drawbacks. It solves the problem of how to design an osteosynthetic clamp for attaching a pedicle screw or spinal hook to a spinal support rod with an adjustable distance between the central axis of the pedicle screw and the central axis of the support rod, and at the same time permitting angular adjustment of the central axis of the pedicle screw relative to the central axis of the support rod.

The invention solves the problem with a clamp comprising the features of claim 1 and a fixation assembly comprising the features of claim 16.

In a preferred embodiment the clamp may comprise two sections adapted to receive the head of a pedicle screw, a hook which holds the support rod at an adjustable distance from the pedicle screw and compression means which holds the two sections together so that they tightly clamp the head of the pedicle screw.

In a preferred embodiment the invention comprises a clamp having a jaw with upper and lower sections, hinged at one end, said sections being bifurcated to form a slot extending through the sections, a socket formed in the jaw for receiving the head of a pedicle screw and compression means movable relative to said socket for forcing the jaws together, said compression means being adapted to receive a support rod and being operable to urge said rod against the jaw as the jaw sections are forced together.
In another embodiment the invention comprises a clamp having a jaw with an upper section and a shorter section, hinged at one end, said upper section being bifurcated to form a slot extending through it, a socket formed in the jaw for receiving the head of a pedicle screw, receiving means which receives a support rod and holds the support rod against the lower surface of the upper section, and compression means, such as a screw extending through the upper and lower sections, which forces them together so as to grasp the head of the pedicle screw.

In a preferred embodiment the invention comprises a clamp having front and back section which form a socket for the pedicle screw head, the front section having a bifurcated extension. The clamp further comprises receiving means which receives a support rod and compression means, such as a screw which holds the front and back sections together, grasping the head of the pedicle screw.
In a preferred embodiment the invention includes a fixation assembly comprising a clamp as described, a pedicle screw having a head shaped to engage the socket and a support rod.

In a preferred embodiment the invention comprises clamp having a front and a back section forming one single block, the front section having a hollow-cylindrical bore for receiving the support rod and the back section having a slot for adjustably receiving the head region of a pedicle screw.

In a preferred embodiment the invention comprises a clamp having a front and a back section forming one single element, the front section being bifurcated to form a slot extending through it, the back section forming a spherical head for rotatable fixation in the head region of a pedicle screw.

The advantages offered by the invention are mainly the following:
- ease of manipulation for the surgeon;
- adaptability of the system, due to the fact that each pedicle screw may be placed at a different distance from the support rod; and
- possibility to use not only deformable support rods, but also rigid support rods which offer an increased mechanical strength.

The various features of novelty which characterize the invention are pointed out with particularity in the claims annexed to and forming part of this disclosure. For the better understanding of the invention, its operating advantages and specific objects attained by its use, reference should be had to the accompanying drawings and descriptive matter in which are illustrated and described preferred embodiments of the invention.

In the drawings:
Fig. 1 is a side elevational view of a fixation assembly comprising a clamp according to the invention showing the support rod in cross-section;
Fig. 2 is a plan view of the fixation assembly of Fig. 1;
Fig. 3 is a side view of the fixation assembly of Figs. 1 and 2, partially cut away to show the entire head of the pedicle screw;
Fig. 4 is a side view of a fixation assembly comprising a preferred embodiment of the invention;
Fig. 5 is a plan view of the clamp and pedicle screw of Fig. 4;
Fig. 6 is a perspective view of a fixation assembly comprising a preferred embodiment of the invention;
Fig. 7 is a top view of the clamp of Fig. 6;
Fig. 8 is a top view of a partial fixation assembly comprising a preferred embodiment of the invention;
Fig. 9 is plan view of the clamp of the assembly of Fig. 8;
Fig. 10 is a side view of the clamp of the assembly of Fig. 8;
Fig. 11 is a frontal sectional view through the pedicle screw of the clamp of the assembly of Fig. 8;
Fig. 12 is a lateral sectional view through the pedicle screw of the clamp of the assembly of Fig. 8;
Fig. 13 shows the fixation of the clamp of the assembly of Fig. 12 in the head region of the pedicle screw of Fig. 12;
Fig. 14 is a lateral view of the partial assembly of Fig. 13.
Fig. 15 is an exploded view of a preferred embodiment of a fixation assembly according to the invention;
Fig. 16 is plan view of the clamp of the assembly of Fig. 15;
Fig. 17 is a side view of the clamp of the assembly of Fig. 15;
Fig. 18 is a top view of the hook of the assembly of Fig. 15;
Fig. 19 is a side view of the hook of the assembly of Fig. 15; and
Fig. 20 is a perspective view of the assembly of Fig. 15.

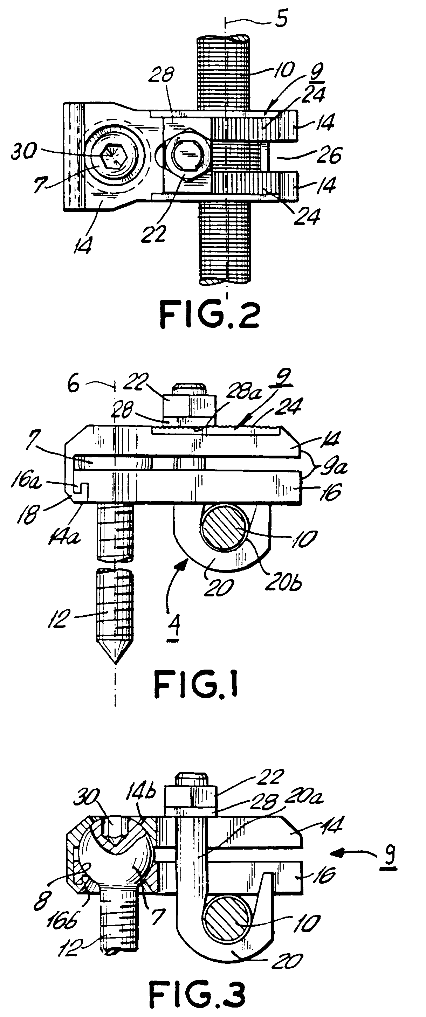

As shown in Fig. 1, a clamp 9 according to the invention holds a support rod 10, which is preferably threaded, and a pedicle screw 12 in a fixed position, more or less perpendicular to each other. The clamp 1 comprises a jaw 9a having an upper section 14 and a lower section 16, connected to each other by a C-shaped extension 14a of the upper section 14 which engages a flange 16a of the lower section 16 to form a hinge 18. Towards their ends near hinge 18, upper and lower sections 14 and 16 are shaped to form a socket 8 (as represented in Fig. 3) adapted to accommodate the head 7 of the pedicle screw 12. In preferred embodiment of the assembly comprising a clamp according to the invention as shown in Fig. 3, the pedicle screw 12 has an essentially spherical head 7. The upper section 14 of jaw 9a has an aperture 14b providing access to the screw head 7. Lower section 16 has an aperture 16b to accommodate the shaft of the pedicle screw 12.

The surface of the head 7 of pedicle screw 12 may be roughened to provide better grip by the clamp 9. The inner surfaces of the socket 8 which holds the head 7 may also be roughened.

Compression means are provided to press the upper and lower sections 14 and 16 of the jaw 9a together. As shown in Figs. 1 to 3 the compression means may be a hook 20. As shown in Fig. 2 and 3, the upper and lower sections 14 and 16 are bifurcated to provide a slot 26. The shaft 20a of hook 20 passes through this slot 26. the bight 20b of hook 20 forms a space under lower section 16 adapted to receive a support rod 10. The inner surface of bight 20b may be threaded or otherwise roughened to engage threads or a roughened surface on support rod 10. The lower surface of lower section 16 may be threaded, knurled or otherwise roughened in the area where it contacts support rod 10, in order to engage threads or the like on the support rod 10. The textured surfaces provide for a better grip on the support rod 10, which must be held firmly in place.
The upper surface of upper section 14 has toothed areas 24 along the sides of through slot 26. A small retaining plate 28 with teeth 28a on one surface rests on top of and bridges the toothed areas 24, with the two sets of teeth interlocking. The distance between the axis of shaft 20a of hook 20 and the central axis of pedicle screw 12 can be varied by moving the small toothed plate 28 along the length of through slot 26. Nut 22 is threaded on the hook shaft 20a on top of plate 28. Tightening nut 22 locks plate 28 and hook 20 into the desired place along through slot 26.

In using the device according to the invention, the pedicle screw 12 is first run through the aperture 16b in the lower jaw section 16 and inserted into the bone, using the hexagonal socket 30 in the head 7 of the pedicle screw 12 to receive a suitable tool. The upper section 14 is then engaged with lower section 16 to form a jaw 9a. Hook 20 is loosely inserted in through slot 26 and the support rod 10 inserted in the bight 20b of the hook 20, the hook 20 being moved along the through slot 26 to the desired position. When pedicle screw 12 and support rod 10 are at the optimum distance from one another and at the proper angle, nut 22 is turned down on the shaft 20a of the hook 20, forcing the jaw sections 14 and 16 together to clamp the head 7 of the pedicle screw 12 in this socket 8 and press support rod 10 firmly against lower section 16. The leverage provided by the jaw construction 9a enables the pedicle screw 12 to be tightly fixed in its selected position relation to the support rod 10.

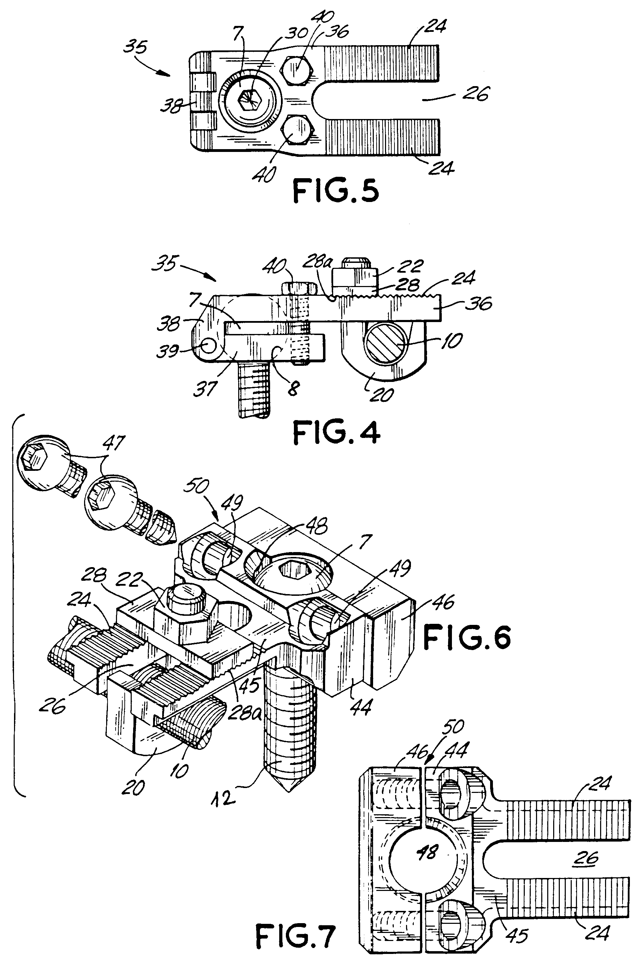

Another embodiment of the invention is shown in Figs. 4 and 5. Clamp 35 comprises a bifurcated upper section 36 and a lower section 37, connected to each other by a hinge 38 with a pin 39. On one side of pedicle screw 12 opposite the hinge 38 are two threaded screws 40. These screws hold upper section 36 and lower section 37 together, thus locking the head 7 of pedicle screw 12 in place. Fig. 5 shows two screws 40, but in alternative embodiment, a single screw 40 may be used.

Lower section 37 is shorter than upper section 36, as shown in Fig. 4. A hook 20 as described in connection with Figs. 1 - 3 passes through the slot 26 formed by the bifurcation of upper section 36 and holds a support rod 10 against the lower surface of upper section 36. It should be evident that the configuration of the toothed upper surface 24, small retaining plate 28 and nut 22 described in connection with Figs. 1 - 3 are applicable to this embodiment as well.
The manner of use of the embodiment according to Figs. 4 and 5 is parallel to that according to Figs. 1 - 3.

Another embodiment of the invention is shown in Figs. 6 and 7. In this embodiment the head 7 of the pedicle screw 12 instead of being clamped by two hinged elements is held between two sections of a block which are joined by screws or bolts.
Referring to Fig. 6, the clamp in this embodiment comprises a block 50 having a front section 44 and a back section 46. Front section 44 has a bifurcated extension 45 which forms a slot 26. As shown in Fig. 6 a hook 20, support rod 10, toothed upper surface 24, small toothed plate 28 and nut 22 are provided as described above in connection with Figs. 1 - 5. Front section 44 has two screw holes 49 through it. As shown in Fig. 6, these screw holes 49 are at an angle of about 45° to the top surface of the clamp or the axis of the pedicle screw 12 to be retained in the clamp. These screw holes 49 continue into back section 46 at the same angle. In a preferred embodiment, only the parts of screw holes 49 which are in the back section 46 are threaded, while screws 47 may be partially or wholly threaded.

Front and back sections 44 and 46 are shaped to form a socket 48 which accommodates the head 7 of a pedicle screw 12. Engagement of screws 47 into the threaded holes in back section 46 forces the back section 46 against front section 44 and secures pedicle screw 12 in its desired position.

When using this embodiment of the invention, the pedicle screw 12 is first inserted into the bone. Front section 44 is placed on the front of the screw head 7, with a hook 20 inserted in slot 26 and the support rod 10 held in the bight of the hook 20. When the pedicle screw 12 and the support rod 10 are adjusted to the optimum distance and angle, back section 46 is placed on the back of the pedicle screw head 7. Screws 47 are inserted into screw holes 49 and tightened. In the embodiment shown in Fig. 6, screws 47 have hexagonal holes in their heads to receive a tool for tightening them.
The 45° degree angles make it easier for the surgeon to reach the screws 47. In an alternative configuration (not shown) the screws 47 are put in straight from back section 46 to front section 44, with the axis of the screw holes 49 parallel to the bifurcated extension 45 or generally perpendicular to the axis of the pedicle screw 12.
The ends of extension 45 may be flanged as shown in Fig. 6, or they may have a simple rectangular cross-section, on any of the embodiments of the invention.

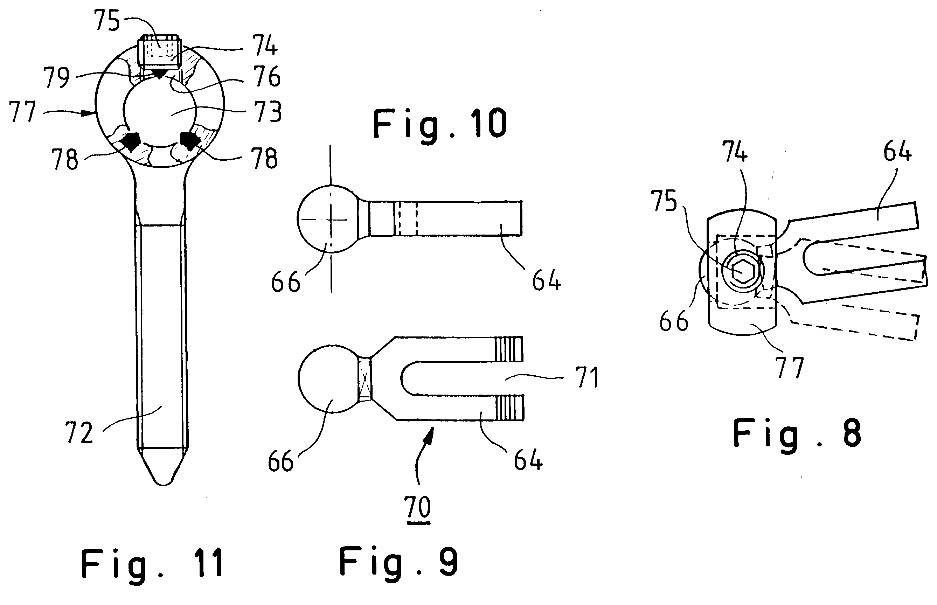

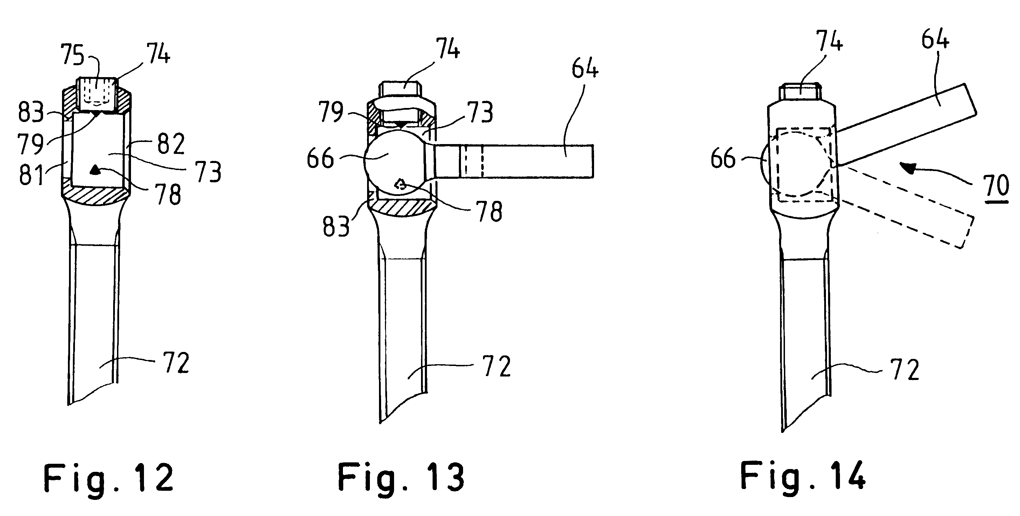

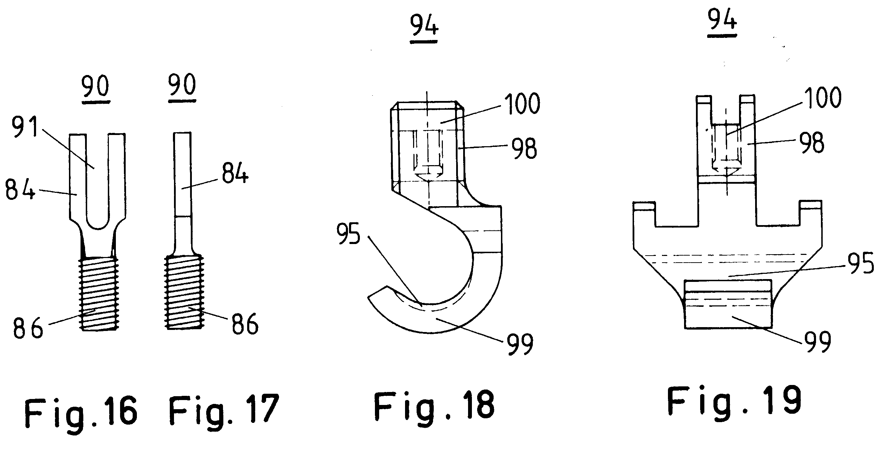

Another embodiment of the invention is shown in Figs. 8 to 14. In this embodiment the clamp 70 is similar to clamp 35 of Fig. 5; the difference being that the head of the pedicle screw is not clamped by suitable elements of the clamp but is incorporated in the clamp 70 itself for being fixed into the head of the pedicle screw.
Clamp 70 therefore has a front section 64 and a back section 66 forming one single element, the front section 64 being bifurcated - in the same way as clamp 35 of Fig. 5 - to form a slot 71 extending through it, the back section 66 forming a spherical head for rotatable fixation in the head region 77 of a pedicle screw 72.
The head region 77 of the pedicle screw 72 has a frontally accessible socket 73 into which the spherical head of the back section 66 of the clamp 70 can be inserted and secured by means of the central screw 74. Central screw 74 can be tightened by means of the central hexagonal bore 75 into a corresponding screw hole 76 in the head region 77. Fixation of the spherical head 66 in the socket 73 occurs by means of two anchoring points incorporated in the lower half of the head region 77 and third anchoring point 79 incorporated in the lower surface of the central screw 74. Socket 73 has circular back opening 81 and a circular front opening 82, said back opening 81 having a diameter inferior to the diameter of the spherical head 66 and said front opening 82 having a diameter superior to the diameter of the spherical head 66. By this construction the spherical head 66 can be safely fixed in the socket 73 - as shown in Fig. 17 - by means of the points 78,79 against the annular rim 83 of back opening 81.
As shown in Fig. 14 this embodiment of the invention allows the angular adjustment of the clamp 70 with relation to the pedicle screw 72.
Fixation of the support rod 10 is achieved in the same way as for clamp 35 of Fig. 4 by means of suitable hook 20 as described in connection with Figs. 1 - 3 which passes through the slot 71 formed by the bifurcation of front section 64 and holds a support rod 10 against its lower surface. It should be evident that the configuration of the toothed upper surface 24, small retaining plate 28 and nut 22 described in connection with Figs. 1 - 3 are applicable to this embodiment as well.

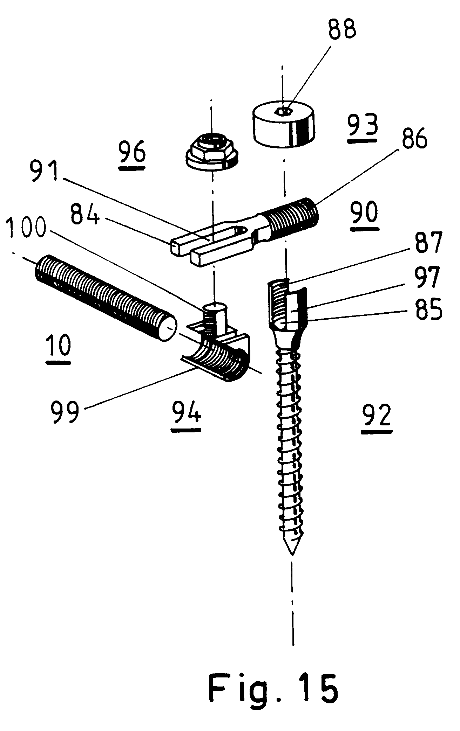

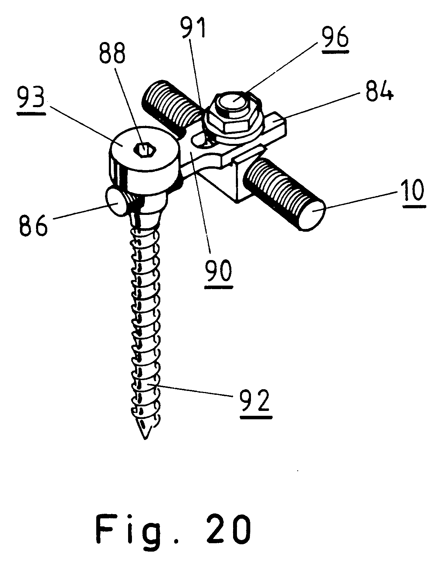

Still another embodiment of the invention is shown in Figs. 15 to 20. In this embodiment clamp 90 is somewhat similar to clamp 70 of Fig. 9; the difference being that the bifurcated front section 84 is not connected to a spherical head but to a longitudinal cylindrical section which is provided with threads.
Clamp 90 therefore has a front section 84 and a back section 86 forming one single element, the front section 84 being bifurcated - in the same way as clamp 70 of Fig. 9 - to form a slot 91 extending through it, the back section 86 forming a longitudinal cylindrical section 86 which is provided with threads for fixation in the head region 97 of a pedicle screw 92.

The head region 97 of pedicle screw 92 is also bifurcated forming a canal 85 for receiving the back section 86 of clamp 90, which can be secured by means of the screw cap 93. Screw cap 93 has preferably a (not-represented) coaxial inner cylindrical portion with reduced diameter which is threaded at its surface for engagement into the inner thread 87 of the bifurcated head region 97 of the pedicle screw 92. Screw cap 93 may be fastened to the head region 97 by means of a hexagonal screw driver engaging with the hexagonal bore 88 of screw cap 93.
Fixation of the support rod 10 is achieved in a similar way as for clamp 35 of Fig. 4 by means of a suitable hook 94 as described in connection with Figs. 1 - 3. The hook 94 has a threaded portion 95 in the inner concavity of its curved portion 99 and an outer threaded portion 98 on its straight portion 100. The straight portion 100 of the hook 94 passes through the slot 91 formed by the bifurcation of front section 84 and upon fixation with the nut 96 on the threaded portion 98 holds a support rod 10 against its lower threaded surface 95. It should be evident that the configuration of the toothed upper surface 24, small retaining plate 28 and nut 22 described in connection with Figs. 1 - 3 are applicable to this embodiment as well.

The various surfaces described in connection with Figs. 1 - 3, as being roughened to provide better grip may of course be employed in the other embodiments too.

From a consideration of the foregoing description it will be evident that a clamp according to the invention permits movement of the support rod relative to the pedicle screw. The surgeon can therefore regulate the horizontal distance between the pedicle screw and the support rod. Because the spherical head of the pedicle screw may be tilted in its socket the angle between the pedicle screw and the support rod may also be adjusted. Thus the clamp according to the invention permits the surgeon to adjust each pedicle screw to the specific configuration required by a particular patient.

Although the invention has been described as applied to a pedicle screw, it is clearly also applicable to other similar devices such as spinal hooks.

## Claims

1. An implantable, unilateral osteosynthetic clamp (9;35) for attaching a single pedicle screw (12) or spinal hook to a single spinal support rod (10) wherein the central axis (6) of said pedicle screw (12) or spinal hook and the central axis (5) of said spinal support rod (10) are located in different planes, and wherein the inclination of said two axis (5,6) is variable relative to one another, and wherein means are provided allowing the adjustable clamping of said pedicle screw (12) or spinal hook with respect to said spinal support rod (10) such that the distance of said two axis (5,6) relative to one another is adjustable, characterised by said means comprising a through slot (26), receiving means (4) adapted to receive the spinal support rod (10), said receiving means (4) being arranged movably within said through slot (26) and compression means (22) for clamping the pedicle screw (12) relative to said receiving means (4).

2. Clamp according to claim 1, characterised in that it comprises:
a first section (14) and a second section (16), at least one of said section (14) being bifurcated to form a through slot (26),
a socket (8) formed in said first and second sections (14,16) for receiving the head (7) of a pedicle screw (12),
receiving means (4) adapted to receive a support rod (10), said receiving means (4) being arranged movably within said through slot (26) relative to said socket (8), and compression means (22) for forcing said two sections (14,16) together to clamp the head (7) of a pedicle screw (12) lodged in said socket (8).

3. Clamp according to claim 1, characterised in that it comprises:
a jaw having an upper section (36) and a lower section (37), said lower section being preferably shorter than said upper section (36), hingedly (38,39) connected to each other, preferably at one of their ends, one of said sections (36,37) at least being bifurcated to form a slot (26) extending through at least on of said sections (36,37),
a socket (8) formed in said jaw for receiving the head (7) of a pedicle screw (12),
first compression means (22) movable in said slot relative to said socket (8), said compression means (22) being adapted to receive a support rod (10) and to urge said support rod (10) against the slotted section (36) of said jaw; and
second compression means (40), for forcing the sections (36,37) of said jaw together to grasp the head (7) of a pedicle screw (12) lodged in said socket (8).

4. Clamp according to claim 1, characterised in that it comprises:
a front section (44) and a back section (46) forming a block (50), said front section (44) being provided with a through slot (26), preferably forming a bifurcation of said front section (44),
a socket (48) formed in said front and back sections (44,46) for receiving the head (7) of a pedicle screw (12),
receiving means (4) adapted to receive and clamp a support rod (10), said receiving means (4) being movable in said slot relative to said socket (8), and
compression means comprising one or more screws (47) adapted to fit into one or more screw holes (49) extending from one section (44) into the other section (46) for forcing said two sections (44,46) together to grasp the head (7) of a pedicle screw (12) lodged in said socket (8).

5. Clamp according to claim 1, characterised in that it comprises:
a front section (64) and a back section (66), said front section (64) being bifurcated to form a through slot (71) and said back section (66) being of spherical shape for fixation into the head region (77) of a pedicle screw (72),
receiving means (4) adapted to receive and clamp a support rod (10), said receiving means (4) being movable in said slot relative to said front section (64), and
compression means (74) for fixing said spherical back section (66) within the head region (77) of a pedicle screw (72).

6. Clamp according to claim 2 or 5, characterised in that the receiving means (4) comprises a hook (20) having a bight (20b) and a shaft (20a) extending through the slot (26) in said section or sections (14,16), said bight (20b) being adapted to receive a support rod (10).

7. Clamp according to claim 6, characterised in that it comprises further a retaining plate (28) on the upper surface of one section (14), said retaining plate (28) having a hole to receive the shaft (20a) of the hook (20).

8. Clamp according to claim 7, characterised in that it comprises further a nut (22) on the end of the shaft (20a) of the hook (20) for holding the retaining plate (28) against the upper surface of one section (14).

9. Clamp according to one of the claims 6 to 8, characterised in that the retaining plate (28) and the upper surface of one section (14) have toothed areas (28a,24) adapted to engage one another.

10. Clamp according to one of the claims 2 to 4, characterised in that the socket (8) is adapted to receive a spherical-headed pedicle screw (12).

11. Clamp according to one of the claims 2 to 4, characterised in that the socket (8) has roughened surfaces.

12. Clamp according to one of the claims 6 to 9, characterised in that a surface of the bight (20b) of the hook (20) is threaded for engagement with threads of a support rod (10).

13. Clamp according to one of the claims 2 to 12, characterised in that one of said sections (16;46) has a roughened surface for contact with a support rod (10).

14. Clamp according to claim 4, characterised in that said screw holes (49) extend from the front section (44) into the back section (46) at an angle of approximately 45° to the axis (6) of the pedicle screw (12).

15. Clamp according to claim 1, characterised in that it comprises:
a front section (84) and a back section (86) forming one single element, the front section 84 being provided with a through slot (91), preferably forming a bifurcation of said front section (84), and said back section (86) being of longitudinal cylindrical shape for fixation into the head region (87) of a pedicle screw (92),
receiving means (94) adapted to receive a support rod (10), compression means (93) for fixing said longitudinal cylindrical back section (86) within the head region (87) of a pedicle screw (92); and
compression means (96) for fixing said support rod (10) inserted in said receiving means (94) relative to said front section (84).

16. Fixation assembly with a clamp (9;35) according to one of the claims 1 to 15, characterised in that it comprises further
a support rod (10); and
a pedicle screw (12).

17. Fixation assembly according to claim 16 characterised in that the pedicle screw (12) has a spherical head (7).

## Patentansprüche

1. Implantierbare, einseitige, osteosynthetische Klemme (9, 35) zur Befestigung einer einzigen Pedikelschraube (12) oder eines Wirbelsäulehakens an einen einzigen Wirbelsäule-Stützstift (10), wobei die Mittelachse (6) der Pedikelschraube (12) oder des Wirbelsäulehakens und die Mittelachse (5) des Wirbelsäule-Stützstifts (10) in unterschiedlichen Ebenen liegen, die Neigung der beiden Achsen (5, 6) zueinander einstellbar ist und Glieder zur einstellbaren Klemmung der Pedikelschraube (12) oder des Wirbelsäulehakens an den Stützstift (10) vorgesehen sind, so daß der Abstand der beiden Achsen (5, 6) zueinander einstellbar ist, dadurch gekennzeichnet, daß die Glieder einen durchgehenden Schlitz (26), Aufnahmeglieder (4) zur Aufnahme des Wirbelsäule-Stützstifts (10), die Aufnahmeglieder (4), welche verschiebbar in dem durchgehenden Schlitz (26) eingesetzt sind, und Glieder (22) zur Klemmung der Pedikelschraube (12) an die Glieder (4) umfassen.

2. Klemme gemäß Patentanspruch 1, dadurch gekennzeichnet, daß sie folgendes umfaßt:
ein erstes Teil (14) und ein zweites Teil (16), von denen wenigstens eins (14) gegabelt ist und einen durchgehenden Schlitz (26) bildet,
eine Fassung (8), die durch das genannte erste und zweite Teil (14, 16) entsteht und den Kopf (7) der Pedikelschraube (12) aufnimmt,
Aufnahmeglieder (4) zur Aufnahme eines Stützstifts (10), wobei die Aufnahmeglieder (4) beweglich zur Fassung (8) in dem durchgehenden Schlitz (26) eingesetzt sind, und Preßglieder (22) zum Zusammenpressen der beiden Teile (14, 16), damit diese den Kopf (7) der in genannter Fassung (a) befindlichen Pedikelschraube (12) festklemmen.

3. Klemme gemäß Patentanspruch 1, dadurch gekennzeichnet, daß sie folgendes umfaßt:
eine Klemmbacke mit einem oberen Teil (36) und einem unteren Teil (37), von denen das untere Teil vorzugsweise kürzer als das genannte obere Teil (36) sein sollte, wobei die beiden Teile über ein Gelenk (38, 39) miteinander verbunden sind, vorzugsweise an einem ihrer Enden, und wobei wenigstens eins der Teile (36, 37) gegabelt ist, um einen Schlitz (26) zu bilden, der wenigstens durch eins der Teile (36, 37) führt,
eine Fassung (8), die durch die genannte Klemmbacke zur Aufnahme des Kopfes (7) einer Pedikelschraube (12) entsteht,
ersten Preßglieder (22), die beweglich zu genannter Fassung (8) in dem Schlitz eingesetzt sind, wobei die Preßglieder (22) einen Stützstift (10) aufnehmen und den Stützstift (10) gegen den geschlitzten Teil (36) der Klemmbacke pressen können, und
zweiten Preßglieder (40), um die Teile (36, 37) der Klemmbacke zusammenzupressen und den Kopf (7) einer in genannter Fassung (8) befindlichen Pedikelschraube (12) aufzunehmen.

4. Klemme gemäß Patentanspruch 1, dadurch gekennzeichnet, daß sie folgendes umfaßt:
ein Vorderteil (44) und ein Rückteil (46), die einen Block (50) darstellen, wobei das Vorderteil (44) einen durchgehenden Schlitz (26) aufweist, der vorzugsweise durch Gabelung des Vorderteils (44) entsteht,
eine im Vorderteil und Rückteil (44, 46) gebildete Fassung (48) zur Aufnahme des Kopfes (7) einer Pedikelschraube (12),
Aufnahmeglieder (4) zur Aufnahme und Klemmung eines Stützstifts (10), wobei die Aufnahmeglieder (4) beweglich zu genannter Fassung (8) in dem Schlitz eingesetzt sind, und
Preßglieder, die eine oder mehrere Schrauben (47) umfassen, die in eines oder mehrere Schraublöcher (49) passen, die sich von einem Teil (44) bis in das andere Teil (46) erstrecken, um die beiden Teile (44, 46) zusammenzupressen und den Kopf (7) einer in genannter Fassung (8) befindlichen Pedikelschraube (12) einzufassen.

5. Klemme gemäß Patentanspruch 1, dadurch gekennzeichnet daß sie folgendes umfaßt:
ein Vorderteil (64) und ein Rückteil (66), wobei das genannte Vorderteil (64) gegabelt ist, um einen durchgehenden Schlitz (71) zu bilden, und das genannte Rückteil (66) kugelförmig ist, um im Kopfteil (77) einer Pedikelschraube (72) befestigt zu werden,
Aufnahmeglieder (4) zur Aufnahme und Klemmung eines Stützstifts (10), wobei die Aufnahmeglieder (4) beweglich zu dem Vorderteil (64) in dem Schlitz eingesetzt sind, und sowie Preßglieder (74) zur Befestigung des kugelförmigen Rückteils (66) im Kopfteil (77) einer Pedikelschraube (72).

6. Klemme gemäß Patentanspruch 2 oder 5, dadurch gekennzeichnet, daß die Aufnahmeglieder (4) einen Haken (20) mit Buchtung (20b) und einen Schaft (20a) durch den Schlitz (26) in dem Teil oder Teilen (14, 16) umfassen, wobei die genannte Buchtung (20b) einen Stützstift (10) aufnehmen kann.

7. Klemme gemäß Patentanspruch 6, dadurch gekennzeichnet, daß sie zudem eine Halterungsplatte (28) auf der Obenfläche eines Teils (14) umfaßt, wobei die genannte Halterungsplatte (28) ein Loch zur Aufnahme des Schaftes (20a) des Hakens (20) aufweist.

8. Klemme gemäß Patentanspruch 7, dadurch gekennzeichnet, daß sie zudem eine Schraubenmutter (22) am Ende des Schaftes (20a) des Hakens (20) zum Festhalten der Halterungsplatte (28) gegen die Obenfläche eines Teils (14) aufweist.

9. Klemme gemäß einem der Patentansprüche 6 bis 8, dadurch gekennzeichnet, daß die Halterungsplatte (28) und die Obenfläche eines Teils (14) gezahnte Bereiche (28a, 24) aufweisen, die ineinandergreifen.

10. Klemme gemäß einem der Patentansprüche 2 bis 4, dadurch gekennzeichnet, daß die Fassung (8) eine Pedikelschraube (12) mit kugelförmigem Kopf aufnehmen kann.

11. Klemme gemäß einem der Patentansprüche 2 bis 4, dadurch gekennzeichnet, daß die Fassung (8) aufgerauhte Oberflächen aufweist.

12. Klemme gemäß einem der Patentansprüche 6 bis 9, dadurch gekennzeichnet, daß eine Fläche der Buchtung (20b) des Hakens (20) gewindet ist, um in das Gewinde eines Stützstifts (10) einzugreifen.

13. Klemme gemäß einem der Patentansprüche 2 bis 12, dadurch gekennzeichnet, daß eines der Teile (16, 46) eine aufgerauhte Oberfläche zur Verbesserung des Kontaktes mit einem Stützstift (10) aufweist.

14. Klemme gemäß Patentanspruch 4, dadurch gekennzeichnet, daß sich die Schraublöcher (49) von dem Vorderteil (44) bis in das Rückteil (46) in einem Winkel von etwa 45° zur Achse (6) der Pedikelschraube (12) erstrecken.

15. Klemme gemäß Patentanspruch 1, dadurch gekennzeichnet, daß sie folgendes umfaßt:
ein Vorderteil (84) und ein Rückteil (86), die ein einziges Element darstellen, wobei das Vorderteil (84) einen durchgehenden Schlitz (91) aufweist, der vorzugsweise durch Gabelung des Vorderteils (84) entsteht, und wobei das genannte Rückteil (86) eine zylinderförmige Längsform zur Befestigung im Kopfteil (87) einer Pedikelschraube (92) aufweist,
Aufnahmeglieder (94) zur Aufnahme eines Stützstifts (10), Preßglieder (93) zur Befestigung des zylinderförmigen länglichen Rückteils (86) im Kopfteil (87) einer Pedikelschraube (92) und
Preßglieder (96) zur Befestigung des Stützstifts (10), der in die Aufnahmeglieder (94) eingesetzt wird, in bezug zu dem Vorderteil (84).

16. Befestigungsvorrichtung mit einer Klemme (9, 35) gemäß einem der Patentansprüche 1 bis 15, dadurch gekennzeichnet, daß sie zudem folgendes umfaßt:
einen Stützstift (10) und
eine Pedikelschraube (12).

17. Befestigungsvorrichtung gemäß Patentanspruch 16, dadurch gekennzeichnet, daß die Pedikelschraube (12) einen kugelförmigen Kopf (7) aufweist.

## Revendications

1. Pince unilatérale implantable pour ostéosynthèse (9; 35) destinée à fixer une vis à pédicule unique (12) ou un crochet vertébral à une tige de support vertébral unique (10), dans laquelle l'axe central (6) de ladite vis à pédicule (12) ou du crochet vertébral et l'axe central (5) de ladite tige de support vertébral (10) sont situés dans des plans différents, et dans laquelle l'inclinaison desdits deux axes (5, 6) peut être modifiée l'un par rapport à l'autre, et dans laquelle des moyens sont prévus qui permettent le pinçage ajustable de ladite vis à pédicule (12) ou du crochet vertébral par rapport à ladite tige de support vertébral (10), de telle sorte que la distance desdits deux axes (5, 6) soit ajustable l'une par rapport à l'autre, caractérisée en ce que lesdits moyens comportent une fente (26), un moyen de réception (4) adapté pour recevoir la tige de support vertébral (10), ledit moyen de réception (4) étant agencé de manière déplaçable dans ladite fente (26), et un moyen de compression (22) pour pincer la vis à pédicule (12) sur ledit moyen de réception (4).

2. Pince selon la revendication 1, caractérisée en ce qu'elle comporte:
une première partie (14) et une seconde partie (16), au moins une desdites parties (14) étant fourchue de manière à former une fente (26),
une douille (8) formée dans ladite première et ladite seconde section (14, 16) pour recevoir la tête (7) d'une vis à pédicule (12),
un moyen de réception (4) adapté pour recevoir une tige de support (10), ledit moyen de réception (4) étant agencé de manière déplaçable dans ladite fente (26) par rapport à ladite douille (8), et un moyen de compression (22) pour repousser ensemble lesdites deux sections (14, 16), pour pincer la tête (7) d'une vis à pédicule (12) logée dans ladite douille (8).

3. Pince selon la revendication 1, caractérisée en ce qu'elle comporte:
une mâchoire présentant une section supérieure (36) et une section inférieure (37), ladite section inférieure étant de préférence plus courte que ladite section supérieure (36), reliées l'une à l'autre par des charnières (38, 39), de préférence à l'une de leurs extrémités, au moins une desdites sections (36, 37) étant fourchue pour former une fente (26) qui traverse au moins une desdites sections (36, 37),
une douille (8) formée dans ladite mâchoire pour recevoir la tête (7) d'une vis à pédicule (12),
un premier moyen de compression (22) pouvant se déplacer dans ladite fente par rapport à ladite douille (8), ledit moyen de compression (22) étant adapté pour recevoir une tige de support (10) et pour presser ladite tige de support (10) contre la section fendue (36) de ladite mâchoire; et
un second moyen de compression (40) pour pousser ensemble les sections (36, 37) de ladite mâchoire, pour saisir la tête (7) d'une vis à pédicule (12) logée dans ladite douille (8).

4. Pince selon la revendication 1, caractérisée en ce qu'elle comporte:
une section avant (44) et une section arrière (46) formant un bloc (50), ladite section avant (44) étant munie d'une fente (26) qui forme de préférence une bifurcation de ladite section avant (44),
une douille (48) formée dans ladite section avant et dans ladite section arrière (44, 46), pour recevoir la tête (7) d'une vis à pédicule (12),
un moyen de réception (4) adapté pour recevoir et pincer une tige de support (10), ledit moyen de réception (4) pouvant être déplacé par rapport à ladite douille (8) dans ladite fente, et
un moyen de compression comportant une ou plusieurs vis (47) adaptées pour s'ajuster dans un ou plusieurs trous pour vis (49) s'étendant d'une section (44) dans l'autre section (46), pour pousser les deux sections ensemble (44, 46), pour saisir la tête (7) d'une vis à pédicule (12) logée dans ladite douille (8).

5. Pince selon la revendication 1, caractérisée en ce qu'elle comporte:
une section avant (64) et une section arrière (66), ladite section avant (64) étant fourchue pour former une fente (71), et ladite section arrière (66) étant de forme sphérique, pour une fixation dans la région de tête (77) d'une vis à pédicule (72),
un moyen de réception (4) adapté pour recevoir et pincer une tige de support (10), ledit moyen de réception (4) étant mobile par rapport à ladite section avant (64) dans ladite fente, et
un moyen de compression (74) pour fixer ladite section arrière sphérique (66) dans la région de tête (77) d'une vis à pédicule (72).

6. Pince selon la revendication 2 ou 5, caractérisée en ce que le moyen de réception (4) comporte un crochet (20) présentant une anse (20b) et une tige (20a) s'étendant à travers la fente (26) dans ladite section ou lesdites sections (14, 16), ladite anse (20b) étant adaptée pour recevoir une tige de support (10).

7. Pince selon la revendication 6, caractérisée en ce qu'elle comporte en outre une plaque de retenue (28) à la surface supérieure d'une section (14), ladite plaque de retenue (28) présentant un trou pour recevoir la tige (20a) du crochet (20).

8. Pince selon la revendication 7, caractérisée en ce qu'elle comporte en outre un écrou (22) à l'extrémité de la tige (20a) du crochet (20), pour retenir la plaque de retenue (28) contre la surface supérieure d'une section (14).

9. Pince selon l'une quelconque des revendications 6 à 8, caractérisée en ce que la plaque de retenue (28) et la surface supérieure d'une section (14) présentent des régions dentées (28a, 24) adaptées pour s'engager l'une sur l'autre.

10. Pince selon l'une quelconque des revendications 2 à 4, caractérisée en ce que la douille (8) est adaptée pour recevoir une vis à pédicule à tête sphérique (12).

11. Pince selon l'une quelconque des revendications 2 à 4, caractérisée en ce que la douille (8) présente des surfaces rendues rugueuses.

12. Pince selon l'une quelconque des revendications 6 à 9, caractérisée en ce qu'une surface de l'anse (20b) du crochet (20) est filetée pour s'engager sur des filets d'une tige de support (10).

13. Pince selon l'une quelconque des revendications 2 à 12, caractérisée en ce que l'une desdites sections (16; 46) présente une surface rendue rugueuse, destinée à entrer en contact avec une tige de support (10).

14. Pince selon la revendication 4, caractérisée en ce que lesdits trous pour vis (49) s'étendent de la section avant (44) jusque dans la section arrière (46), suivant un angle d'environ 45° par rapport à l'axe (6) de la vis à pédicule (12).

15. Pince selon la revendication 1, caractérisée en ce qu'elle comporte:
une section avant (84) et une section arrière (86) formant un élément unique, la section avant (84) étant munie d'une fente (91) formant de préférence une bifurcation de ladite section avant (84), et ladite section arrière (86) est de forme cylindrique longitudinale, pour fixation dans la région de tête (87) d'une vis à pédicule (92),
un moyen de réception (94) adapté pour recevoir une tige de support (10), un moyen de compression (93) pour fixer ladite section arrière (86) cylindrique longitudinale dans la région de tête (87) d'une vis à pédicule (92); et
un moyen de compression (96) pour fixer ladite tige de support (10) insérée dans ledit moyen de réception (94), par rapport à ladite section avant (84).

16. Unité de fixation comportant une pince (9; 35) selon l'une quelconque des revendications 1 à 15, caractérisée en ce qu'elle comporte en outre
une tige de support (10); et
une vis à pédicule (12).

17. Unité de fixation selon la revendication 16, caractérisée en ce que la vis à pédicule (12) présente une tête sphérique (7).
